# EUROPEAN PATENT APPLICATION

(11) **EP 4 751 755 A1**
(43) Date of publication of application: **03.06.2026**
(21) Application number: 24876930.9
(22) Date of filing: 16.08.2024
(51) Int. Cl.: A61M 25/10, A61B 17/3205, A61B 17/3209

(54) **BALLOON CATHETER AND METHOD FOR MANUFACTURING BALLOON CATHETER**

(30) Priority: 12.10.2023 JP 2023177056
(71) Applicant: Goodman Co., Ltd., Aichi 460-0008 (JP)
(72) Inventor: OKAMOTO, Mitsumasa, Seto-shi, Aichi 489-0976 (JP); KUNISADA, Takashi, Seto-shi, Aichi 489-0976 (JP)
(74) Representative: Prüfer & Partner mbB Patentanwälte · Rechtsanwälte
(86) International application number: PCT/JP2024/029172
(87) International publication number: WO 2025/079330

(57) **Abstract**

A balloon catheter includes an inflatable and deflatable balloon 13. The balloon 13 includes a straight tube portion 13c in a cylindrical shape with a diameter that is at a maximum when the balloon 13 is inflated. The straight tube portion 13c includes an external protrusion 25 that protrudes from an outer periphery 23 of the straight tube portion 13c. The external protrusion 25 extends in a direction that is angled to an axial direction of the straight tube portion 13c and along the outer periphery 23 of the straight tube portion 13c.

## Description

### Cross-Reference to Related Application

The present application is based on Japanese Patent Application No. 2023-177056 filed on October 12, 2023, the entire contents of which are incorporated herein by reference.

### Technical Field

The present disclosure relates to a balloon catheter and a method for producing the balloon catheter.

### Background Art

A balloon catheter includes an inflatable and deflatable balloon on a distal side of the balloon catheter. The balloon catheter is for dilation of a section of a blood vessel that is narrowed or blocked with a lesion by inserting the balloon that is in a deflated state into the section with the lesion and then by inflating the balloon.

A balloon catheter may include a linear element on an outer periphery of a balloon. The element extends in an axial direction of the balloon. The element protrudes from the outer periphery of the balloon. The balloon catheter can create cracks in the lesion with the element that cuts into the lesion when the balloon is inflated in the section with the lesion. The section with the lesion can be easily dilated using the cracks as a trigger.

As described in PLT1 below, the element is on an outer periphery of a straight tube portion that is in a cylindrical shape and has a diameter that is at a maximum when the balloon is inflated. In a configuration described in PLT1, the element extends in an axial direction of the straight tube portion, that is, parallel to the axial direction.

### Citation List

### Patent Literature

PLT1: International Publication WO2020/255923

### Summary of Invention

### Technical Problem

During the dilation of the section with the lesion with the balloon, the balloon may be moved to different positions with respect to the axial direction in the section with the lesion and inflated for multiple times. The element on the straight tube portion of the balloon in PLT1 extends in the axial direction of the straight tube portion. The element contacts the lesion at the same position with respect to the circumferential direction every time when the balloon is inflated. This may cause difficulties in proper dilation of the section with the lesion using the cracks as a trigger.

The present disclosure has been made in view of the above circumstances. A main objective of the present disclosure is to provide a balloon catheter that properly dilates a section with a lesion and a method for producing the balloon catheter.

### Solution to Problem

To solve the problem described above, a balloon catheter in a first aspect of the present disclosure includes a balloon that is inflatable and deflatable. The balloon includes a straight tube portion that is in a cylindrical shape with a diameter that is at a maximum when the balloon in inflated. The straight tube portion includes an external protrusion that protrudes from an outer periphery of the straight tube portion. The external protrusion extends in a direction that is angled to an axial direction of the straight tube portion and along the outer periphery.

In the first aspect, the straight tube portion of the balloon includes the external protrusion that protrudes from the outer periphery of the straight tube portion. The external protrusion extends in the direction that is angled to the axial direction of the straight tube portion. When the balloon is moved in the axial direction in the section with the lesion, a position of the external protrusion relative to the lesion changes in a circumferential direction of the section with the lesion. According to the configuration, a position at which cracks are created in the lesion with the external protrusion changes in the circumferential direction. That is, the cracks are created in the lesion with the external protrusion at different positions with respect to the circumferential direction. Therefore, the section with the lesion is properly dilated using the cracks as a trigger.

In the balloon catheter in the second aspect, an angle of the external protrusion according to the first aspect relative to the axial direction is in a range from 1 to 10 degrees.

If the angle of the external protrusion relative to the axial direction of the straight tube portion is greater, the external protrusion tends to be longer and thus the straight tube portion tends to be thicker. This may results in a reduction in insertability of the balloon during the insertion of the balloon into the human body. In the second aspect, the angle of the external protrusion is in the range from 1 to 10 degrees, which is considered to be a small angle. Therefore, the length of the external protrusion is less likely to increase and thus the straight tube portion is less likely to be thicker. Therefore, even though the external protrusion is configured to extend at an angle, the insertability of the balloon is less likely to decrease.

In the balloon catheter in the third aspect, the balloon according to the second aspect includes a wing that is formed when the balloon is deflated. The wing extends in the axial direction and is disposed to cover the external protrusion from an outer side.

The balloon includes the wing that is formed when the balloon is deflated. The wing extends in the axial direction of the straight tube portion and is disposed to cover the external protrusion from the outer side. In this configuration, the angle of the external protrusion relative to the axial direction of the straight tube portion is in the range from 1 to 10 degrees, which is considered to be a small angle. Therefore, even though the external protrusion is configured to extend at an angle, the wing is easily formed in a shape to cover the external protrusion.

In a balloon catheter in a fourth aspect, the external protrusion according to any one of the first to the third aspects includes two first portions at ends of the external protrusion with respect to a longitudinal direction of the external protrusion and a second portion between the first portions. An angle of the external protrusion relative to the axial direction is greater in the first portions than in the second portion.

To create cracks in the lesion with the external protrusion by inflating the balloon, use of a middle portion of the external protrusion with respect to the longitudinal direction may be considered. If so, end portions of the external protrusion with respect to the longitudinal direction may contact healthy areas that do not include lesions. In light of such a situation, in the fourth aspect, the external protrusion, the angle of which is greater in the first potions than in the second portion, includes the first portions at ends with respect to the longitudinal direction and the second portion between the first portions. According to the configuration, stretch of end sections of the straight tube portion including the first portions in the circumferential direction during inflation of the balloon is suppressed. This reduces expansion of the end sections. Therefore, the first portions of the external protrusion are less likely to contact the healthy areas.

In a balloon catheter in a fifth aspect, the external protrusion according to any one of the first to the third aspects includes two first portions at ends of the external protrusion with respect to a longitudinal direction of the external protrusion and a second portion between the first portions. An angle of the external protrusion relative to the axial direction is less in the first portions than in the second portion.

Because the angle of the external protrusion in the fifth aspect is less in the first portions than in the second portion in the fifth aspect, a pushing force for pushing the balloon into the human body is easily transmitted to the tip-end side.

In a balloon catheter in a sixth aspect, the balloon according to any one of the first to the third aspects includes two tapered portions that are disposed on sides of the straight tube portion with respect to the axial direction to sandwich the straight tube portion and have a diameter that decreases as a distance from the straight tube portion increases. A predefined one of the tapered portions includes an internal protrusion that protrudes from an inner periphery of the predefined one of the tapered portions and extends in the axial direction and along the inner periphery.

The direction in which the external protrusion extends includes an axial direction component of the straight tube portion. Therefore, the stretch of the straight tube portion in the axial direction during the inflation of the balloon is suppressed. In the sixth aspect, the predefined one of the tapered portions includes the internal protrusion. The internal protrusion extends in the axial direction and along the inner periphery of the predefined one of the tapered portions. According to the configuration, stretch of the predefined one of the tapered portions in the axial direction during the inflation of the balloon is suppressed without a reduction in insertability of the balloon.

In a balloon catheter in a seventh aspect, the external protrusion according to the sixth aspect extends for an entire length of the straight tube portion with respect to the axial direction. The internal protrusion extends for an entire length of the predefined one of the tapered portions with respect to the axial direction. A position of the internal protrusion with respect to the circumferential direction of the balloon is equal to a position of an end of the external protrusion on a side on which the predefined one of the tapered portions is located.

In the seventh aspect, the external protrusion and the internal protrusion are continuous. According to the configuration, the stretch of the straight tube portion and the predefined one of the tapered portions in the axial direction during inflation of the balloon is further suppressed.

In a balloon catheter in an eighth aspect, the external protrusion according to any one of the first to the third aspects has a cross section in an inverted V-shape and includes two side faces that are adjacent to each other across a vertex of the inverted V-shape. One of the side faces has a surface roughness greater than a surface roughness of second side face in a tip-end portion of the external protrusion on a tip-end side with respect to the axial direction. The second side face has a surface roughness greater than a surface roughness of the first side face in a base-end portion of the external protrusion on a base-end side with respect to the axial direction.

According to the configuration in the first aspect in which the external protrusion extends at an angle, the balloon may rotate in the circumferential direction during the inflation of the balloon due to contact of the external protrusion with the lesion. In light of such a situation, in the eighth aspect, the surface roughness of first side face of the external protrusion is defined greater than the surface roughness of the second side face in the tip-end portion of the external protrusion with respect to the axial direction. Further, the surface roughness of the second side face of the external protrusion is defined greater than the surface roughness of the first side face in the base-end portion of the external protrusion with respect to the axial direction. According to the configuration, rotations of the balloon to one side in the circumferential direction and to the other side in the circumferential direction are suppressed. That is, the rotations of the balloon during the inflation of the balloon are properly suppressed.

The surface roughness of the second side face is less in the tip-end portion of the external protrusion with respect to the axial direction and the surface roughness of the first side face is less in the base-end portion of the external protrusion with respect to the axial direction. According to the configuration, in comparison to the configuration in which the side faces both have high surface roughness, a sliding resistance during the insertion of the balloon into the human body is reduced. Therefore, the rotations of the balloon during the inflation of the balloon are properly suppressed without a reduction in insertability of the balloon into the human body.

A method for producing a balloon catheter in a ninth aspect is for producing the balloon catheter according to any one of the first to the third aspects. In the method, a parison that is in a tubular shape and a base material of the balloon includes a protrusion on an outer periphery of the parison. The protrusion that is for forming the external protrusion extends in a longitudinal direction of the parison. A mold for producing the balloon includes an internal cavity for forming the balloon. The internal cavity is in an elongated shape corresponding to a shape of the balloon and includes a cavity portion for forming the straight tube portion. The mold includes a periphery wall that defines the cavity portion. The periphery wall includes a groove that extends in a direction that is angled to a longitudinal direction of the internal cavity. The method includes: a twisting step of twisting the parison in a circumferential direction of the parison that is placed in the internal cavity to cause the protrusion to extend in the direction that is angled to the longitudinal direction; and an expanding step of expanding the parison that is twisted in the twisting step in the internal cavity to form an expanded parison body that includes a portion in a balloon shape. In the expanding step, the protrusion is inserted in the groove by expanding the parison to form the external protrusion.

In the ninth aspect, the parison that is placed in the internal cavity of the mold is twisted in the circumferential direction and thus the protrusion of the parison extends in the direction that is angled to the longitudinal direction of the parison (the twisting step). Then, the twisted parison is expanded to form the expanded parison body that includes the portion in the balloon shape (the expanding step). Then, extra portions of the expanded parison body are removed and the balloon is produced.

A portion of the internal cavity of the mold is the cavity portion for forming the straight tube portion. The periphery wall that defines the cavity portion in the mold includes the groove that extends in the direction that is angled to the longitudinal direction of the internal cavity of the mold. In the expanding step, the external protrusion is formed by expanding the parison to cause the protrusion of the parison to be inserted in the groove of the mold. According to the step, the external protrusion that extends in the direction that is angled to the axial direction of the straight tube portion is formed from the protrusion of the parison that extends in the longitudinal direction of the parison (i.e., the axial direction of the parison). The balloon that includes the external protrusion that is angled is produced using a regular parison that is prepared by extrusion (i.e., a parison that has a constant cross section for an entire length in the longitudinal direction).

In the method for producing a balloon catheter in a tenth aspect, the method according to the ninth aspect further includes a stretching step of stretching the parison that is twisted in the twisting step in the longitudinal direction and holding the parison stretched. The expanding step is performed after the stretching step.

In the tenth aspect, the stretching step is performed for stretching the parison that is twisted in the twisting step and holding the parison stretched and the expanding step is performed after the stretching step. According to the method, the parison is expanded while the parison is properly kept twisted, that is, the protrusion of the parison is properly maintained at an angle relative to the longitudinal direction of the parison. Therefore, the balloon that includes the angled external protrusion is properly produced.

In the method for producing a balloon catheter in an eleventh aspect, the periphery wall in the method according to the ninth aspect includes a protruding portion that protrudes inward on an inner peripheral side of the periphery wall and extends in the direction that is angled. The groove is formed in the protruding portion and extends along the protruding portion.

In the ninth aspect, the periphery wall includes the protruding portion that protrudes inward on the inner peripheral side of the periphery wall. The protruding portion extends in the direction that is angled to the internal cavity of the mold. The groove is formed in the protruding portion and extends along the protruding portion. According to the configuration, the protrusion of the parison is properly inserted in the groove during the expansion of the parison. This properly reduces removal of the protrusion from the groove during the expansion of the parison. Therefore, the external protrusion is properly formed.

### Brief Description of Drawings

The above-described object and any other object, features, and advantages of the present disclosure will be further clarified by the following detailed description made with reference to the attached drawings.
[FIG. 1] A schematic overall side view illustrating a configuration of a balloon catheter according to a first embodiment.
[FIG. 2] (a) is a side view illustrating a balloon in an inflated state and therearound; (b) is a cross-sectional view cut along line A-A in FIG. 2(a); (c) is a cross-sectional view cut along line B-B in FIG. 2(a); and (d) is a cross-sectional view cut along line C-C in FIG. 2(a).
[FIG. 3] (a) is a side view illustrating a configuration of the balloon in a deflated state and therearound; and (b) is a cross-sectional view cut along line D-D in FIG. 3(a).
[FIG. 4] (a) is a cross-sectional view illustrating a configuration of a mold; (b) is a cross-sectional view cut along line E-E in FIG. 4(a); (c) is a cross-sectional view cut along line F-F in FIG. 4(a); (d) is a cross-sectional view cut along line G-G in FIG. 4(a); and (e) is a cross-sectional view cut along line H-H in FIG. 4(a);
[FIG. 5] A perspective view illustrating a parison.
[FIG. 6] (a) is a cross-sectional view illustrating a placing step; (b) is a cross-sectional view illustrating a twisting step; and (c) is a cross-sectional view illustrating a stretching step.
[FIG. 7] (a) is a cross-sectional view illustrating an expanding step; (b) is a cross-sectional view cut along line I-I in FIG. 7(a); (c) is a cross-sectional view cut along line J-J in FIG. 7(a); (d) is a cross-sectional view cut along line K-K in FIG. 7(a); and (e) is a cross-sectional view cut along line L-L in FIG. 7(a).
[FIG. 8] is a view for explaining a method for using the balloon catheter.
[FIG. 9] (a) is a cross-sectional view cut along line M-M in FIG. 8(b); and (b) is a cross-sectional view cut along line N-N in FIG. 8(c).
[FIG. 10] (a) is a cross-sectional view illustrating a configuration of a mold in a second embodiment; (b) is a cross-sectional view cut along line O-O in FIG. 10(a); (c) is a cross-sectional view cut along line P-P in FIG. 10(a); (d) is a cross-sectional view cut along line Q-Q in FIG. 10(a); and (e) is a cross-sectional view cut along line R-R in FIG. 10(a).
[FIG. 11] (a) is a cross-sectional view illustrating an expanding step in the second embodiment; (b) is a cross-sectional view cut along line S-S in FIG. 11(a); (c) is a cross-sectional view cut along line T-T in FIG. 11(a); (d) is a cross-sectional view cut along line U-U in FIG. 11(a); and (e) is a cross-sectional view cut along line V-V in FIG. 11(a).
[FIG. 12] A side view illustrating a configuration of a balloon and therearound in another embodiment.

### Description of Embodiments

### [First Embodiment]

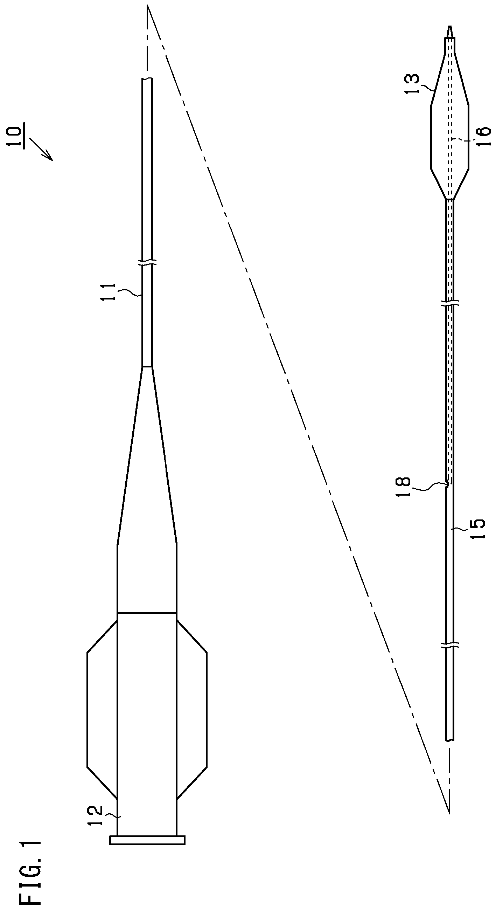

An embodiment of the present disclosure will be described with reference to the drawings. FIG. 1 is a schematic overall side view illustrating a configuration of a balloon catheter.

As illustrated in FIG. 1, a balloon catheter 10 includes a catheter tube 11, a hub 12, and a balloon 13. The hub 12 is mounted to a base-end portion (a proximal end portion) of the catheter tube 11. The balloon 13 is mounted to a tip-end portion (a distal end portion) of the catheter tube 11.

The catheter tube 11 includes an outer tube 15 and an inner tube 16 that is inserted in the outer tube 15. The outer tube 15 is made of a resin material and formed in a tubular shape. A base-end portion of the outer tube 15 is joined to the hub 12. A tip-end portion of the outer tube 15 is joined to the balloon 13. The outer tube 15 has a lumen inside. The lumen of the outer tube 15 is communicated with an internal space of the hub 12 and an internal space of the balloon 13. The lumen of the outer tube 15 is a fluid lumen through which a compressed fluid is passed for inflation and deflation of the balloon 13.

The outer tube 15 may be composed of multiple tubes that are disposed in a row in the axial direction and joined to one another. One of the multiple tubes on a base-end side may be made of a metal material and another of the multiple tubes on a tip-end side may be made of a resin material.

The inner tube 16 is made of a resin material and formed in a tubular shape. A base-end portion of the inner tube 16 is joined to a portion of the outer tube 15 at the middle with respect to the axial direction of the outer tube 15. A portion of the inner tube 16 on the tip-end side extends farther than the tip of the outer tube 15 and inserted in the internal space of the balloon 13. A portion of the inner tube 16 adjacent to the tip-end portion of the inner tube 16 is joined to a tip-end portion of the balloon 13.

The lumen of the inner tube 16 is a guidewire lumen through which a guidewire G is passed. A base opening 18 of the guidewire lumen of the inner tube 16 is located in the middle of the balloon catheter 10 with respect to the axial direction of the balloon catheter 10. That is, the balloon catheter 10 is classified as an RX-type catheter. A base opening 18 may be at a base-end portion of the balloon catheter 10. The balloon catheter 10 in this configuration is classified as an over-the-wire-type catheter.

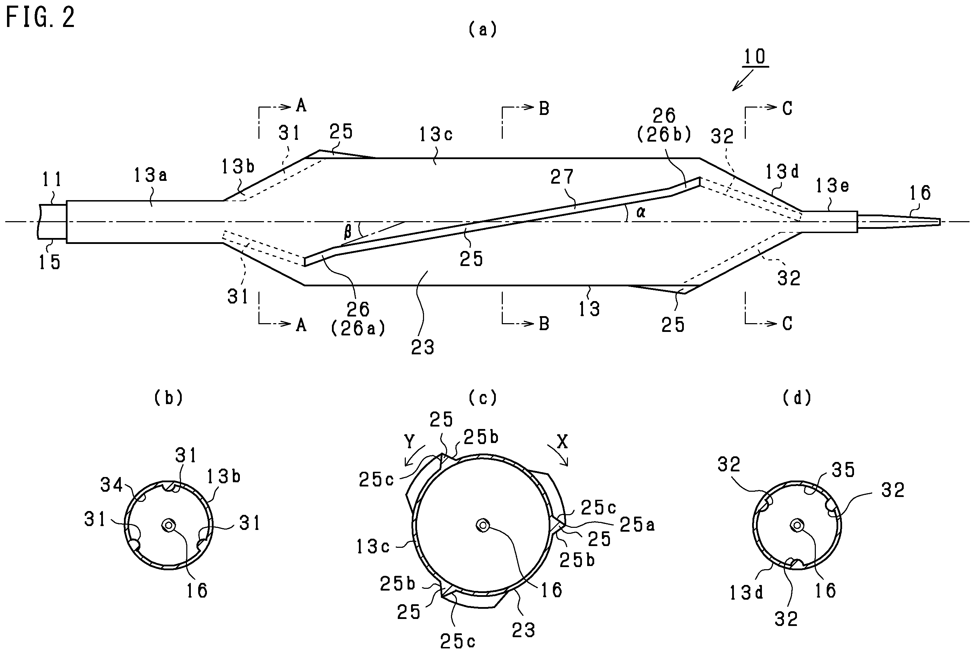

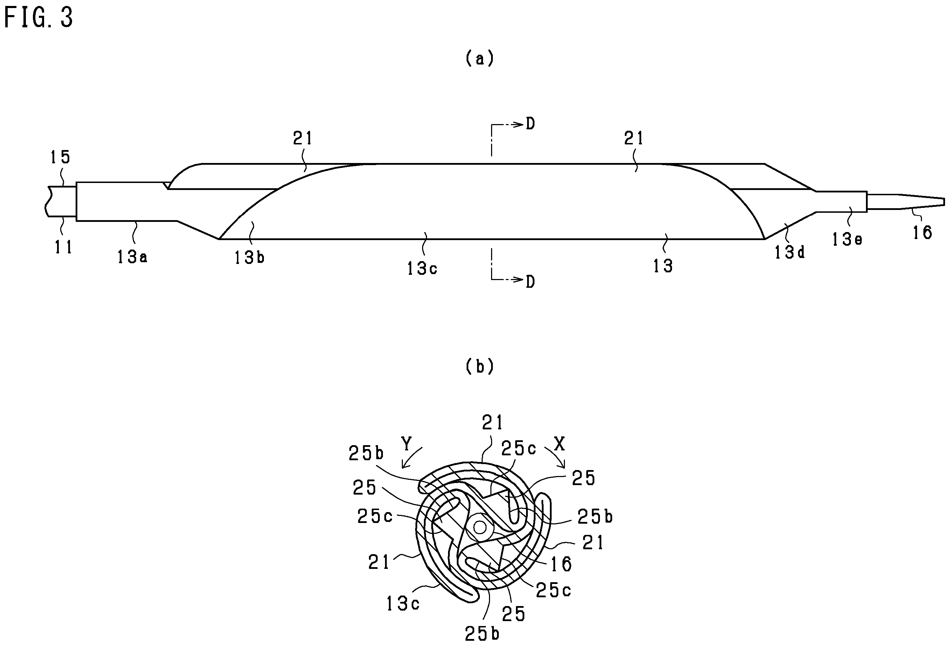

Next, a configuration of the balloon 13 and therearound will be described with reference to FIGS. 2(a) to 3(b). FIG. 2(a) is a side view illustrating the configuration of the balloon 13 in an inflated state and therearound. FIG. 2(b) is a cross-sectional view cut along line A-A in FIG. 2(a). FIG. 2(c) is a cross-sectional view cut along line B-B in FIG. 2(a). FIG. 2(d) is a cross-sectional view cut along line C-C in FIG. 2(a). FIG. 3(a) is a side view illustrating the configuration of the balloon 13 in the deflated state and therearound. FIG. 3(b) is a cross-sectional view cut along line D-D in FIG. 3(a).

The balloon 13 is made of a thermoplastic resin material such as polyamide elastomer. As illustrated in FIG. 2(a), the balloon 13 is formed in a cylindrical shape (a tubular shape) with a circular cross section as a whole. Specifically, the balloon 13 includes a base-end leg portion 13a, a base-end tapered portion 13b, a straight tube portion 13c, a tip-end tapered portion 13d, and a tip-end leg portion 13e. The portions 13a to 13e are arranged in the above sequence from the base-end side toward the tip-end side.

The base-end leg portion 13a is joined to the tip-end portion of the outer tube 15. The base-end tapered portion 13b increases in diameter from the tip of the base-end leg portion 13a toward the tip-end side. That is, the base-end tapered portion 13b is in a tapered shape. The straight tube portion 13c extends from the tip of the base-end tapered portion 13b toward the tip-end side with a constant diameter. That is, the straight tube portion 13c is in a circular tube shape (a cylindrical shape). The straight tube portion 13c has a diameter that is at a maximum when the balloon 13 is inflated. The tip-end tapered portion 13d decreases in diameter from the front end of the straight tube portion 13c toward the tip-end side. That is, the tip-end tapered portion 13d is in a tapered shape. The tip-end leg portion 13e is joined to a portion of the inner tube 16 on the tip-end side. The base-end tapered portion 13b and the tip-end tapered portion 13d correspond to "two tapered portions."

When the compressed fluid is supplied to the internal space of the balloon 13 via the lumen of the outer tube 15, the balloon 13 inflates. When the negative pressure is applied to the lumen of the outer tube 15 and the compressed fluid is discharged from the internal space of the balloon 13, the balloon 13 deflates. As illustrated in FIGS. 3(a) and 3(b), the balloon 13 includes multiple (specifically, three) wings 21 that are formed when the balloon 13 is deflated. The wings 21 are at equal intervals in the circumferential direction of the balloon 13. Each wing 21 extends in the axial direction over the tapered portions 13b, 13d and the straight tube portion 13c of the balloon 13. The wings 21 are folded along the outer periphery of the balloon 13 and wound around the inner tube 16.

The balloon catheter 10 includes linear external protrusions 25 that protrude from an outer periphery 23 of the straight tube portion 13c of the balloon 13. The external protrusions 25 are for creating cracks in a lesion during dilation of a section with the lesion by inflating the balloon 13. According to the balloon catheter 10, even if the lesion is hardened due to calcification or other reasons, the cracks are created in the lesion with the external protrusions 25 and the balloon catheter 10 enables easy dilation of the section with the lesion using the cracks as a trigger of the dilation in which the lesion may be broken. That is, the balloon catheter 10 is classified as a balloon catheter having a scoring function.

A configuration of the external protrusions 25 will be described. As described in FIGS. 2(a) and 2(c), the external protrusions 25 extend in a direction that is angled to the axial direction of the straight tube portion 13c (i.e., the axial direction of the balloon 13) and along the outer periphery 23 of the straight tube portion 13c. Each external protrusion 25 extends from one side with respect to the circumferential direction of the straight tube portion (hereinafter referred to as a first side X) to another side (hereinafter referred to as a second side Y) as approaching from the base-end side to the tip-end side with respect to the axial direction. The first side X and the second side Y are opposite to each other in the circumferential direction of the straight tube portion 13c. The external protrusions 25 extend for an entire length of the straight tube portion 13c with respect to the axial direction.

Multiple external protrusions 25 are at predefined intervals (specifically, equal intervals) in the circumferential direction of the straight tube portion 13c (i.e., the circumferential direction of the balloon 13). In this embodiment, three external protrusions 25 are disposed. The external protrusions 25 have the same configuration. The external protrusions 25 are integrally formed with the balloon 13.

Each external protrusion 25 has a cross section (specifically, a cross section perpendicular to a longitudinal direction of the external protrusion 25) in an inverted V-shape, specifically, a triangular shape that protrudes outward in the radial direction of the balloon 13. An end of protruding of the external protrusion 25 forms a vertex 25a. The external protrusion 25 includes side faces 25b, 25c that are adjacent to each other via the vertex 25a. The side face 25b is located on the first side X and the side face 25c is located on the second side Y. The cross section of the external protrusion 25 is not necessarily in the triangular shape and may be in a semicircular shape, a rectangular shape, a pentagonal shape, or other shapes.

An angle of each external protrusion 25 relative to the axial direction of the straight tube portion 13c is in a range from 1 to 10 degrees. Each external protrusion 25 includes two first portions 26 and a second portion 27. The first portions 26 are at ends of the external protrusion 25 with respect to the longitudinal direction (i.e., ends of the straight tube portion 13c with respect to the longitudinal direction), respectively. The second portion 27 is between the first portions 26. The angle of the external protrusion 25 in the first portions 26 is α. The angle of the external protrusion 25 in the second portion 27 is β that is greater than α (α < β). The second portion 27 takes a large part of the external protrusion 25 with respect to the longitudinal direction. In this embodiment, a length of the second portion 27 is more than five times greater than the length of each first portion 26. That is, the length of the first portion 26 is 1/5 of the length of the second portion 27. The first portions 26 correspond to "first portions" recited in claim 4. The second portion 27 corresponds to "a second portion" recited in claim 4.

The external protrusions 25 that are adjacent to each other in the circumferential direction of the straight tube portion 13c are away from each other in the circumferential direction when viewed in the axial direction of the straight tube portion 13c. According to the configuration, the straight tube portion 13c includes non-protrusion areas in which the external protrusions 25 are not present for an entire length of the straight tube portion 13c with respect to the longitudinal direction. In the straight tube portion 13c, the external protrusions 25 and the non-protrusion areas are alternately disposed in the circumferential direction of the straight tube portion 13c.

The first portions 26 include a first portion 26a and a first portion 26b. The first portion 26a is on the base-end side of the external protrusion 25 with respect to the axis direction. The first portion 26b is on the tip-end side of the external protrusion 25 with respect to the axial direction. In the first portion 26a, a surface roughness of the side face 25c is greater than a surface roughness of the side face 25b. In the first portion 26b, a surface roughness of the side face 25b is greater than a surface roughness of the side face 25c. The surface roughness of the side face 25c in the first portion 26a and the surface roughness of the side face 25b in the first portion 26b are equal. The first portion 26a corresponds to "a base-end portion of the external protrusion on a base-end side with respect to the axial direction." The first portion 26b corresponds to "a tip-end portion of the external protrusion on a tip-end side with respect to the axial direction." The side face 25b corresponds to "a first side face" and the side face 25c corresponds to "a second side face."

In this description, "the surface roughness" means arithmetic mean roughness Ra defined in JIS B0601:2001. The arithmetic mean roughness Ra is measured according to the JIS B0633:2001 and a measuring device defined in JIS B0651:2001 is used for the measurement.

As described earlier, the balloon 13 includes multiple wings 21 that are formed when the balloon 13 is deflated. As illustrated in FIGS. 3(a) and 3(b), the wings 21 are folded in the circumferential direction of the balloon 13, specifically, each wing 21 is folded on the second side Y in the circumferential direction. The external protrusions 25 of the straight tube portion 13c and the wings 21 have a one-to-one relationship. The external protrusions 25 are inside the folded wings 21, respectively. When the balloon 13 is in the deflated state, the external protrusions 25 are covered with the respective wings 21 from the outer side.

The base-end tapered portion 13b and the tip-end tapered portion 13d include linear internal protrusions 31, 32, respectively. As illustrated in FIGS. 2(a) and 2(b), the internal protrusions 31 of the base-end tapered portion 13b protrude from an inner surface 34 of the base-end tapered portion 13b and extend in the axial direction of the balloon 13 and along the inner surface 34 of the base-end tapered portion 13b. A position of each internal protrusion 31 with respect to the circumferential direction of the balloon 13 is constant for the entire length with respect to the axial direction. Specifically, the internal protrusions 31 extend for the entire length of the base-end tapered portion 13b with respect to the axial direction. Multiple internal protrusions 31 are at predefined intervals (specifically, equal intervals) with respect to the circumferential direction of the balloon 13. In this embodiment, three internal protrusions 31 are disposed. The internal protrusions 31 are integrally formed with the balloon 13.

Positions of the internal protrusions 31 are equal to positions of the base ends of the external protrusions 25, respectively, with respect to the circumferential direction of the balloon 13. Each internal protrusion 31 has a cross section (specifically, a cross section perpendicular to the longitudinal direction of the internal protrusions 31) in a semicircular shape that protrudes inward in the balloon 13. The cross section of the internal protrusion 31 is not necessarily in the semicircular shape and may be in a semicircular shape, a rectangular shape, a pentagonal shape, or other shapes.

The internal protrusions 32 of the tip-end tapered portion 13d protrude from the inner surface of the tip-end tapered portion 13d and extend in the axial direction of the balloon 13 and along the inner surface 35 of the tip-end tapered portion 13d. A positon of each internal protrusion 32 with respect to the circumferential direction of the balloon 13 is constant for an entire length with respect to the axial direction. Specifically, the internal protrusions 32 extend for an entire length of the tip-end tapered portion 13d. The internal protrusions 32 are at predefined intervals (specifically, equal intervals) with respect to the circumferential direction of the balloon 13. In this embodiment, three internal protrusions 32 are disposed. The internal protrusions 32 are integrally formed with the balloon 13.

Positions of the internal protrusions 32 are equal to positions of the tip ends of the external protrusions 25, respectively, with respect to the circumferential direction of the balloon 13. Each internal protrusion 32 has a cross section (specifically, a cross section perpendicular to the longitudinal direction of the internal protrusion 32) in a semicircular shape that protrudes inward in the balloon 13. The cross section of the internal protrusion 32 is not necessarily in the semicircular shape and may be in a triangular shape, a rectangular shape, a pentagonal shape, or other shapes.

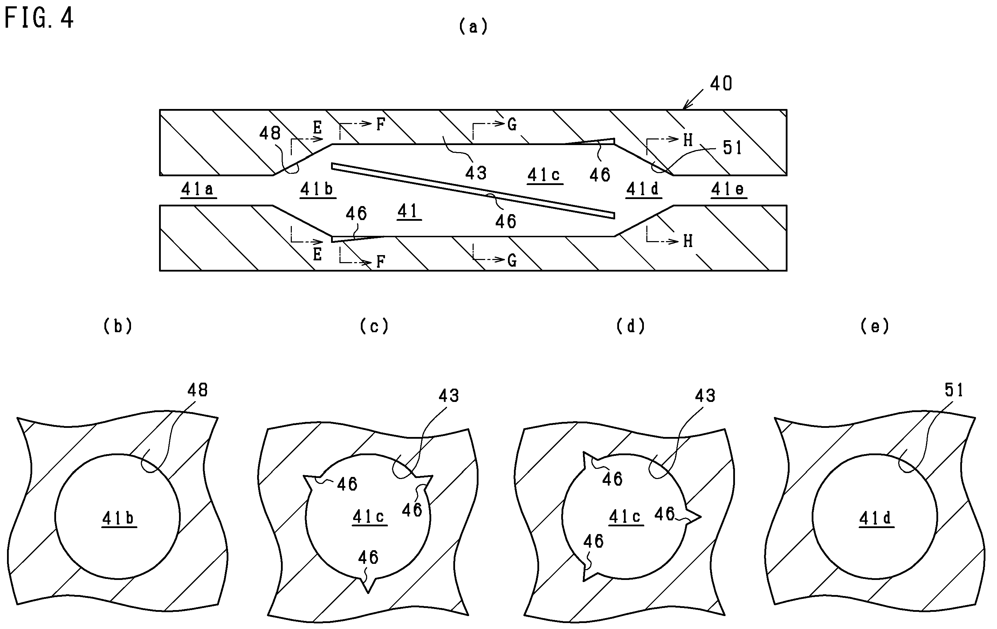

Next, a method for producing the balloon 13 that is described above will be described. The balloon 13 is manufactured using a mold 40. First, a configuration of the mold 40 will be described with reference to FIGS. 4(a) to 4(e). FIG. 4(a) is a cross-sectional view illustrating the configuration of the mold 40. FIG. 4(b) is a cross-sectional view cut along line E-E in FIG. 4(a). FIG. 4(c) is a cross-sectional view cut along line F-F in FIG. 4(a). FIG. 4(d) is a cross-sectional view cut along line G-G in FIG. 4(a). FIG. 4(e) is a cross-sectional view cut along line H-H in FIG. 4(a).

As illustrated in FIG. 4(a), the mold 40 has a cuboid shape and includes an internal cavity 41 inside for forming the balloon 13. The internal cavity 41 has a longitudinal shape that extends in the longitudinal direction of the mold 40 to correspond to the shape of the balloon 13. As illustrated in FIGS. 4(b) to 4(e), the internal cavity 41 has a cross section (specifically, a cross section perpendicular to the longitudinal direction of the internal cavity 41) in a circular shape for the entire length with respect to the longitudinal direction.

The internal cavity 41 includes a cavity portion 41a, a cavity portion 41b, a cavity portion 41c, a cavity portion 41d, and a cavity portion 41e. The cavity portion 41a is for forming the base-end leg portion 13a of the balloon 13. The cavity portion 41b is for forming the base-end tapered portion 13b of the balloon 13. The cavity portion 41c is for forming the straight tube portion 13c of the balloon 13. The cavity portion 41d is for forming the tip-end tapered portion 13d of the balloon 13. The cavity portion 41e is for forming the tip-end leg portion 13e of the balloon 13. The cavity portions 41a to 41e have shapes that correspond to the portions 13a to 13e of the balloon 13. The cavity portion 41c for the straight tube portion 13c corresponds to "a cavity portion" recited in the claims.

As illustrated in FIGS. 4(c) and 4(d), the mold 40 includes a periphery wall 43 that defines the cavity portion 41c for the straight tube portion 13c. The periphery wall 43 includes multiple (specifically, three) grooves 46. Each groove 46 extends in a direction that is angled to the longitudinal direction of the internal cavity 41 and opens to the cavity portion 41c. The grooves 46 are at equal intervals in the circumferential direction of the periphery wall 43. The grooves 46 in the periphery wall 43 extend for an entire length of the internal cavity 41 with respect to the longitudinal direction of the internal cavity 41. Each groove 46 has a cross section (specifically, a cross section perpendicular to the longitudinal direction of the groove 46) in a triangular shape. The cross section of the groove 46 is not necessarily in the triangular shape and may be in a rectangular shape, a pentagonal shape, a semicircular shape, or other shapes.

As illustrated in FIG. 4(b), the mold 40 includes a periphery wall 48 that defines the cavity portion 41b for the base-end tapered portion 13b. The cavity portion 41b includes a tapered cavity corresponding to the base-end tapered portion 13b. As illustrated in FIG. 4(e), the mold 40 includes a periphery wall 51 that defines the cavity portion 41d for the tip-end tapered portion 13d. The cavity portion 41d includes a tapered cavity corresponding to the tip-end tapered portion 13d.

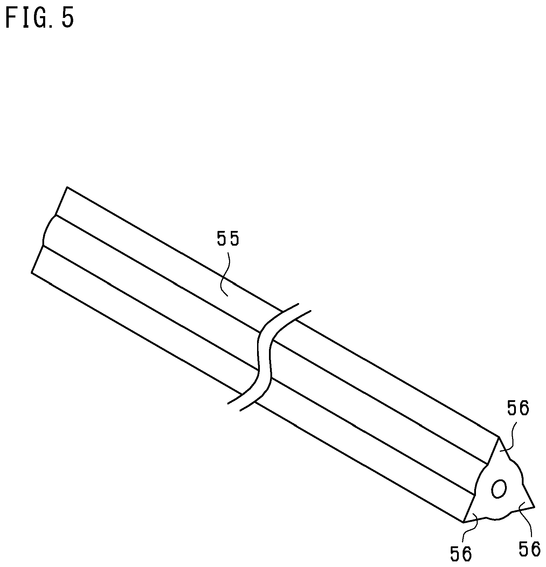

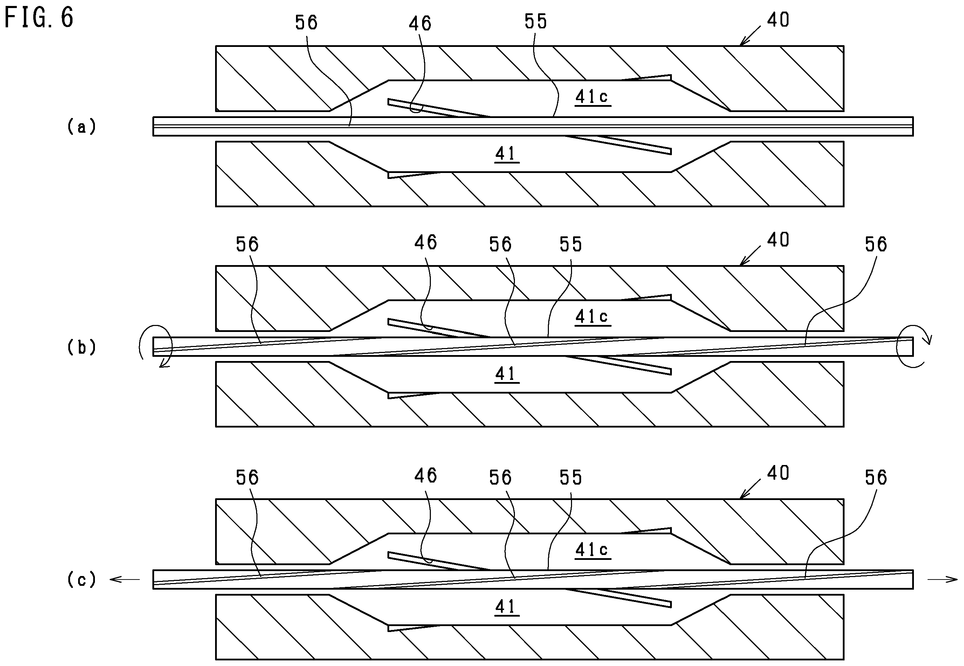

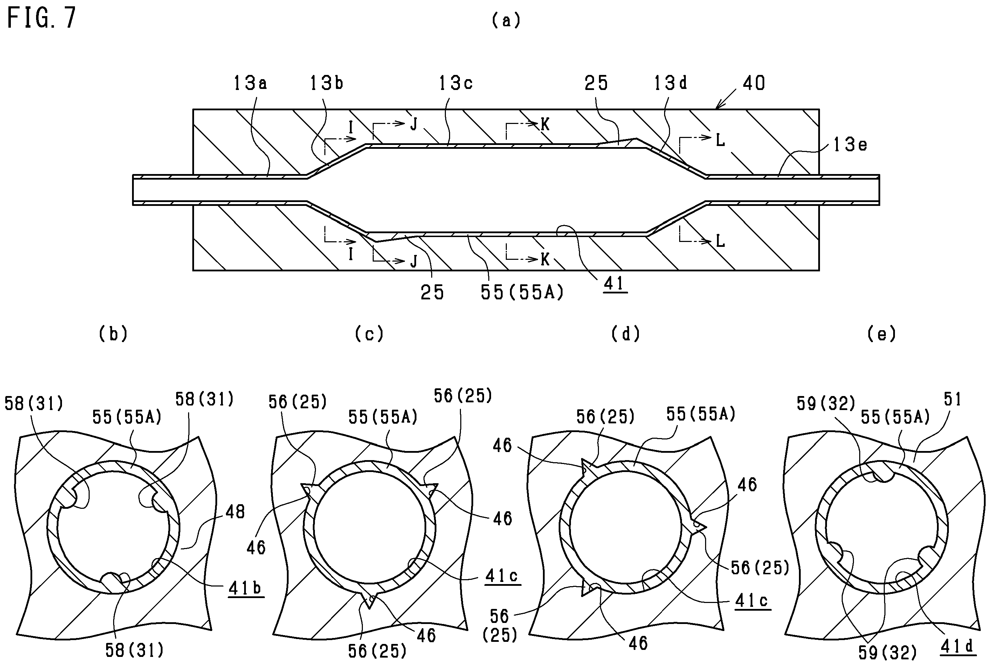

Next, the method for producing the balloon 13 using the mold 40 described above will be described with reference to FIGS. 5 to 7(e). FIG. 5 is a perspective view of a parison 55. FIG. 6(a) is a cross-sectional view illustrating a placing step. FIG. 6(b) is a cross-sectional view illustrating a twisting step. FIG. 6(c) is a cross-sectional view illustrating a stretching step. In FIGS. 6(a) to 6(c), the parison 55 is depicted in a side view. FIG. 7(a) is a cross-sectional view illustrating an expanding step. FIG. 7(b) is a cross-sectional view cut along line I-I in FIG. 7(a). FIG. 7(c) is a cross-sectional view cut along line J-J in FIG. 7(a). FIG. 7(d) is a cross-sectional view cut along line K-K in FIG. 7(a). FIG. 7(e) is a cross-sectional view cut along line L-L in FIG. 7(a).

To produce the balloon 13, first, a step for preparing the mold 40 and the parison 55 is performed. The balloon 13 is produced from the parison 55. As illustrated in FIG. 5, the parison 55 may be made of a resin and formed in a round tubular shape by extrusion. The parison 55 includes multiple (specifically, three) protrusions 56 that protrude from an outer periphery. Each protrusion 56 has a cross section (a cross section perpendicular to the longitudinal direction of the parison 55) in an inverted V-shape (specifically, a triangular shape) that protrudes outward in the radial direction of the parison 55. The protrusions 56 extend in the longitudinal direction of the parison 55 for an entire length of the parison 55. The protrusions 56 are for forming the external protrusions 25 and the internal protrusions 31, 32 of the balloon 13. The cross section of each protrusion 56 is not necessarily in the triangular shape and may be in a rectangular shape, a pentagonal shape, a semicircular shape, or other shapes.

Next, the placing step illustrated in FIG. 6(a) is performed for placing the parison 55 in the internal cavity 41 of the mold 40. In this step, the parison 55 is placed in the internal cavity 41 with the longitudinal direction of the parison 55 aligned with the longitudinal direction of the internal cavity 41. Through the step, the parison 55 is placed across the cavity portions 41a to 41e.

Next, the twisting step illustrated in FIG. 6(b) is performed for twisting the parison 55 in the circumferential direction to cause each protrusion 56 of the parison 55 to extend in a direction that is angle to the longitudinal direction of the parison 55. In this step, ends of the parison 55 with respect to the longitudinal direction of the parison 55 are rotated in opposite directions in the circumferential direction of the parison 55 to twist the parison 55 in the circumferential direction. Specifically, the ends of the parison 55 with respect to the longitudinal direction are fixed to the fixtures and rotated in opposite directions to twist the parison 55 in the circumferential direction.

Alternatively, to twist the parison 55 in the circumferential direction, only one of the fixtures may be rotated and the other one of the fixtures may be held so as not to rotate. The parison 55 may be twisted by rotating one of the fixtures relative to the other one of the fixtures.

Next, the stretching step illustrated in FIG. 6(c) is performed for stretching the parison 55 that has been twisted in the twisting step and holding the parison 55 stretched. In this step, the fixtures are moved away from each other to stretch the parison 55 in the longitudinal direction. Through this step, the parison 55 is properly held twisted.

Next, the expanding step illustrated in FIG. 7(a) is performed for forming an expanded parison body 55A that includes portions that are to be formed for portions of the balloon 13 by thermally expanding the parison 55 in the twisted state as described above in the internal cavity 41 of the mold 40. In this step, the parison 55 is expanded by inserting a fluid such as nitrogen into the parison 55. Through this step, the expanded parison body 55A that includes the portions 13a to 13e of the balloon 13 is prepared.

In the expanding step, the external protrusions 25 are formed by expanding the parison 55 and inserting the protrusions 56 of the parison 55 into the grooves 46 of the mold 40 as illustrated in FIGS. 7(c) and 7(d). Namely, the sections of the protrusions 56 inserted in the grooves 46 form the external protrusions 25.

In the expanding step, the protrusions 56 of the parison 55 are placed in the grooves 46 along the grooves 46. Because the parison 55 is held twisted, a restoring force is exerted on the parison 55 in the opposite direction to the twisting direction. With restoring force, the parison 55 is placed with portions in the grooves 46 pressed against sidewalls of the grooves 46. Specifically, portions of the parison 55 in the grooves 46 located at ends of the grooves 46 with respect to the longitudinal direction (hereinafter, the portions will be referred to as in-groove tip-end portions and in-groove base-end portions) are pressed against the sidewalls of the grooves 46. The in-groove base-end portions are formed into the first portions 26a of the external protrusions 25 and the in-groove tip-end portions are formed into the first portions 26b of the external protrusions 25. More specifically, the in-groove tip-end portions and the in-groove base-end portions are pressed against sidewalls that are different from each other among the sidewalls of the grooves 46. This increases surface roughness of side faces of the in-groove tip-end portions and the in-groove base-end portions. Surface roughness of the side faces 25b in the first portions 26b is increased and surface roughness of the side faces 25c in the first portions 26a are increased.

In the expanding step, as illustrated in FIG. 7(b), the protrusions 56 of the parison 55 are pressed against the periphery wall 48 of the mold 40 and smashed by expanding the parison 55 to cause portions 58 of the parison 55 along the protrusions 56 to protrude inward on an inner peripheral side to form the internal protrusions 31. In the expanding step, as illustrated in FIG. 7(e), through expansion of the parison 55 and smash of the protrusions 56 of the parison 55 by pressing the protrusions 56 against the periphery wall 51 of the mold 40, portions 59 of the parison 55 along the protrusions 56 protrude inward on the inner peripheral side and the internal protrusions 32 are formed.

After the expanding step, a cutting step is performed for cutting extra end portions of the expanded parison body 55A. Through the step, the balloon 13 is produced and the producing of the balloon 13 is complete.

Then, post-processing, which may include a joining step for joining the balloon 13 to the catheter tube 11 and a joining step for joining the hub 12 to the catheter tube 11, is performed. This completes a series of the producing steps.

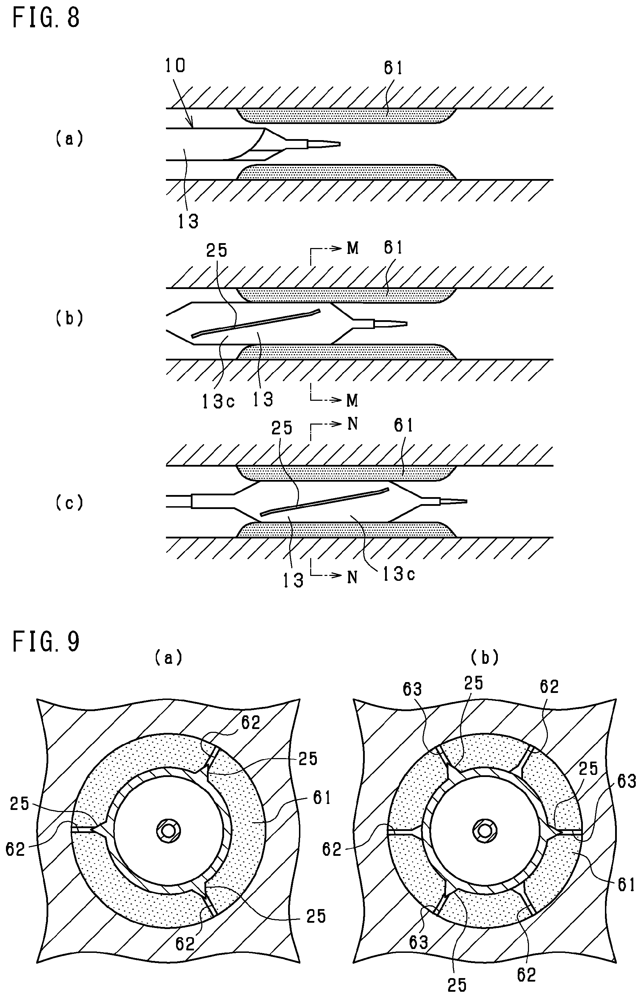

Next, a method of using the balloon catheter 10 will be described. A procedure of dilating a section of a blood vessel with a lesion using the balloon catheter 10 will be described with reference to FIGS. 8(a) to 9(b). FIGS. 8(a) to 8(c) are views illustrating the procedure. FIG. 9(a) is a cross-sectional view cut along line M-M in FIG. 8(b). FIG. 9(b) is a cross-sectional view cut along line N-N in FIG. 8(c).

First, a guiding catheter is inserted into a sheath introducer that is in the blood vessel and then a tip opening of the guiding catheter is inserted into a coronary artery inlet. The guidewire G is then inserted into the guiding catheter and the inserted guidewire G is inserted from the coronary artery inlet to a distal section via the section with the lesion.

Then, the balloon catheter 10 is inserted into the guiding catheter along the guidewire G. After the insertion, pushing force and pulling force are applied until the balloon 13 is placed in a section with a lesion 61. During the insertion, as illustrated in FIG. 8(a), the balloon 13 remains deflated. The lesion 61 is formed in the blood vessel for an entire circumference of the blood vessel.

As illustrated in FIG. 8(b), when the balloon 13 has reached the section with the lesion 61, the balloon 13 is inflated. The external protrusions 25 are pressed against the lesion 61 and cracks 62 are created in the lesion 61 with the external protrusions 25 (see FIG. 9(a)).

Next, the balloon 13 is deflated and moved toward the distal side (the farther side) in the axial direction inside the section with the lesion 61. As illustrated in FIG. 8(c), the balloon 13 is inflated again at the position to which the balloon 13 is moved. The external protrusions 25 are pressed against the lesion 61 and cracks 63 are created in the lesion 61 with the external protrusions 25 (see FIG. 9(b)). The cracks 63 are created at positions different from the positions of the cracks 62 in the circumferential direction of the lesion 61. The cracks 62 are created in the lesion 61 when the balloon 13 is first inflated (at the inflation illustrated in FIG. 8(b)).

Because each external protrusion 25 of the balloon catheter 10 extend in the direction that is angled to the axial direction of the straight tube portion 13c, the position of the external protrusion 25 relative to the lesion 61 changes in the circumferential direction of the lesion 61 as the position of the balloon 13 is changed in the axial direction inside the section with the lesion 61 as described above (see FIGS. 9(a) and 9(b)). When the balloon 13 is inflated for multiple times while the position of the balloon 13 is changed in the axial direction as described above, cracks are created in the lesion 61 with the external protrusion 25 at different positions with respect to the circumferential direction of the lesion 61. With the external protrusions 25, the cracks 62, 63 are created in the lesion 61 at the different positions with respect to the circumferential direction. Therefore, the section with the lesion 61 is properly dilated using the cracks 62, 63 as a trigger of the dilation.

After the dilation of the section with the lesion 61 with the balloon 13 is completed, the balloon 13 is deflated. Then, the balloon catheter 10 is pulled out of the human body while the balloon 13 is kept deflated. This completes a series of the steps.

As described above, the balloon catheter 10 is generally used for blood vessel treatment, for example, treatment for coronary arteries, femoral arteries, pulmonary arteries, or other types of blood vessels; however, the balloon catheter 10 can be used for treatment of "tubes" or "body cavities" in living organism other than blood vessels such as ureters and gastrointestinal tracts.

According to the configurations of the embodiment described in detail above, the following advantageous effects are produced.

The angle of each external protrusion 25 relative to the axial direction of the straight tube portion 13c is in the range from 1 to 10 degrees, which is small. Therefore, the length of the external protrusion 25 is less likely to increase and thus the straight tube portion 13c is less likely to be thicker. Therefore, even though each external protrusion 25 is configured to extend at an angle, the insertability of the balloon 13 is less likely to decrease.

The wings 21 of the balloon 13 extend in the axial direction of the straight tube portion 13c and cover the external protrusions 25 from an outer side. In such a configuration, the angle of each external protrusion 25 relative to the axial direction of the straight tube portion 13 is in the range from 1 to 10 degrees, which is considered to be a small angle, as described above. The external protrusions 25 extend substantially along the respective wings 21. According to such a configuration in which the external protrusions 25 extend at an angle, the winds 21 are easily formed with the external protrusions 25 are covered with the respective wings 21.

For creating cracks in a lesion with the external protrusions 25 by inflating the balloon 13, the cracks may be created with middle portions of the external protrusions 25 with respect to the longitudinal direction. In this case, the ends of the external protrusions 25 with respect to the longitudinal direction may contact healthy areas that do not include lesions. Therefore, in this embodiment, each external protrusion 25 includes two first portions 26 at the ends with respect to the longitudinal direction and the second portion 27 between the first portions 26. The angle of the external protrusion 25 is greater in the first portions 26 than in the second portion 27. According to the configuration, stretch of the end sections of the straight tube portion 13c on which the first portions 26 are disposed in the circumferential direction during the inflation of the balloon 13 is suppressed. Therefore, expansion of the sections is suppressed. The first portions 26 of the external protrusions 25 are less likely to contact the healthy areas.

The direction in which each external protrusion 25 extends includes an axial direction component of the straight tube portion 13c. Therefore, stretch of the straight tube portion 13c in the axial direction during the inflation of the balloon 13 is suppressed. Further, the base-end tapered portion 13b (corresponding to "a predefined one of the tapered portions") of the balloon 13 includes the internal protrusions 31. Each internal protrusion 31 extends in the axial direction and along the inner surface 34 of the base-end tapered portion 13b. According to the configuration, stretch of the base-end tapered portion 13b in the axial direction during the inflation of the balloon 13 is suppressed without a reduction in insertability of the balloon 13.

The external protrusions 25 extend for the entire length of the straight tube portion 13c in the axial direction. The internal protrusions 31 extend for the entire length of the base-end tapered portion 13b in the axial direction. The positions of the internal protrusions 31 with respect to the circumferential direction are equal to the positions of the ends of the respective external protrusions 25 on the base-end tapered portion 13b side (i.e., the base ends of the external protrusions 25). According to the configuration, the external protrusions 25 are continuous from the respective internal protrusions 31. Therefore, the stretch of the straight tube portion 13c and the base-end tapered portion 13b in the axial direction during the inflation of the balloon 13 is further suppressed.

The tip-end tapered portion 13d of the balloon 13 (corresponding to "a predefined one of the tapered portions") includes the internal protrusions 32. The internal protrusions 32 extend in the axial direction and along the inner surface 35 of the tip-end tapered portion 13d. According to the configuration, stretch of the tip-end tapered portion 13d in the axial direction during the inflation of the balloon 13 is suppressed without a reduction in insertability of the balloon 13.

The internal protrusions 32 extend for the entire length of the tip-end tapered portion 13d with respect to the axial direction. The positions of the internal protrusions 32 with respect to the circumferential direction of the balloon 13 are equal to the positions of the ends of the external protrusions 25 on the tip-end tapered portion 13d side (i.e., the tip-end portions of the external protrusion 25). According to the configuration, the external protrusions 25 are continuous from the respective internal protrusions 32. Therefore, the stretch of the straight tube portion 13c and the tip-end tapered portion 13d in the axial direction during the inflation of the balloon 13 is further suppressed.

The first portion 26b on the tip-end portion side of each external protrusion 25 with respect to the axil direction includes the side face 25b with the surface roughness greater than the surface roughness of the side face 25c. The first portion 26a on the base-end portion side of each external protrusion 25 with respect to the axial direction includes the side face 25c with the surface roughness greater than the surface roughness of the side face 25b. According to the configuration, rotation of the balloon 13 in the circumferential direction toward the first side X and rotation of the balloon 13 toward the second side Y are suppressed. Therefore, the rotation of the balloon 13 during the inflation of the balloon 13 is properly suppressed.

The first portion 26b of each external protrusion 25 on the tip end side with respect to the axial direction includes the side face 25c with the less surface roughness. The first portion 26a of each external protrusion 25 on the base end side with respect to the axial direction includes the side face 25b with the less surface roughness. In comparison to a configuration in which the side faces 25b, 25c have greater surface roughness, sliding resistance of the balloon 13 during the insertion of the balloon 13 into the body is reduced. Therefore, the rotation of the balloon 13 during the inflation of the balloon 13 is properly suppressed.

If the balloon 13 has a configuration in which the external protrusions 25 of the balloon 13 that is deflated are covered with wings 21 from the outer side, the external protrusions 25 may not be entirely covered with the wings 21 and ends of the external protrusions 25 may stick out of the wings 21. The above configuration in which either one of the side faces 25c, 25b of each of the first portions 26a, 26b on the ends of each external protrusion 25 has the less surface roughness is effective for reducing the sliding resistance of the balloon 13.

When the balloon 13 is deflated, the wings 21 are folded toward the second side Y with respect to the circumferential direction of the balloon 13 and cover the external protrusions 25 from the outer side. In such a configuration, the side faces 21c located on the second side Y are more likely to be out of the wings 21 in comparison to the side faces 21b located on the first side X. Focusing on such a point, in the first portion 26b of each external protrusion 25 on the tip-end side in the above embodiment, the surface roughness of the side face 25c is less than the surface roughness of the side face 25b. According to the configuration, even if the first portions 26b (side faces 21c) of the external protrusions 25 are out of the wings 21, the sliding resistance during the insertion of the balloon 13 is reduced. Especially, the surface roughness of the side faces 25c of the first portions 26b on the tip-end side among the first portions 26a, 26b of the external protrusions 25 and thus the sliding resistance during the insertion of the balloon 13 is properly reduced.

The parison 55 is twisted in the circumferential direction in the internal cavity of the mold 40 to make the protrusions 56 of the parison 55 extend in the direction that is angled to the longitudinal direction of the parison 55 (the twisting step) and then the expanded parison body 55A that includes the portion in the shape corresponding to the shape of the balloon 13 is formed by expanding the parison 55 that has been twisted (the expanding step). In the expanding step, the protrusions 55 are inserted into the grooves 46 of the mold 40 by expanding the parison 55 to form the external protrusions 25. Through the step, the external protrusions 25 that extend in the direction that is angled to the axial direction of the straight tube portion 13c are formed from the protrusions 56 that extend in the longitudinal direction of the parison 55. According to the step, the balloon 13 that includes the external protrusions 25 that are angled is formed from the parison 55 that is formed by extrusion and has a regular configuration (that is, the parison 55 that has the cross section in the same shape for the entire length in the longitudinal direction).

The stretching step is performed to hold the parison 55 that has been twisted in the twisting step stretched in the longitudinal direction and the expanding step is performed after the stretching step. According to the step, the parison 55 is expanded while properly holding the parison 55 twisted, that is, properly holding the protrusions 56 of the parison 55 angled to the longitudinal direction of the parison 55. Through the steps, the balloon 13 that includes the angled external protrusions 25 are properly formed.

### [Second Embodiment]

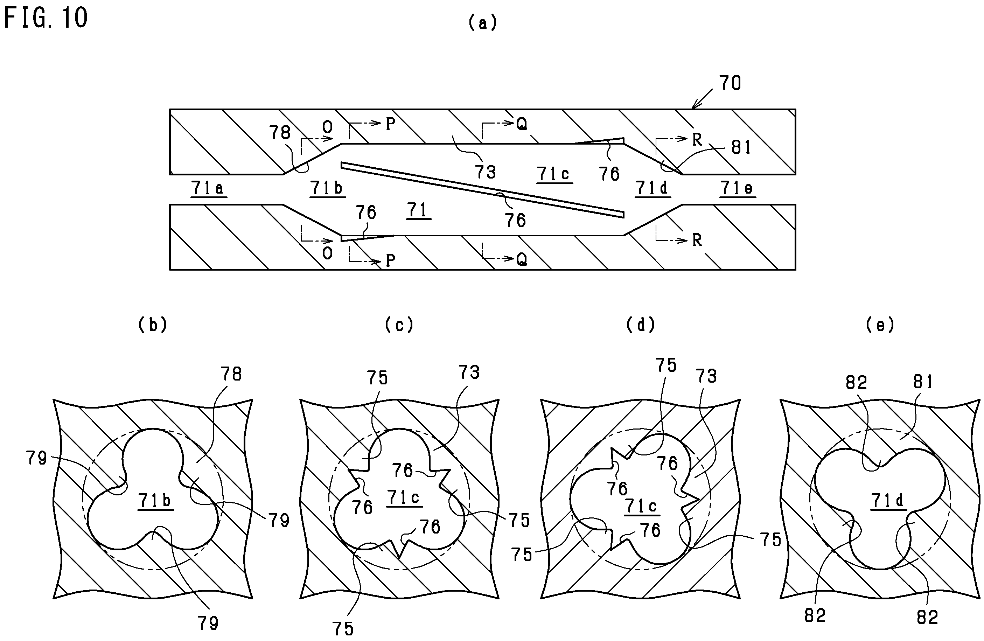

Next, a second embodiment will be described. In this embodiment, a mold that has a configuration different from the configuration of the mold in the first embodiment is used for producing the balloon 13. The configuration of the mold in this embodiment will be described according to FIGS. 10(a) to 10(e). FIG. 10(a) is a cross-sectional view illustrating the configuration of the mold in this embodiment. FIG. 10(b) is a cross-sectional view cut along line O-O in FIG. 10(a). FIG. 10(c) is a cross-sectional view cut along line P-P in FIG. 10(a). FIG. 10(d) is a cross-sectional view cut along line Q-Q in FIG. 10(a). FIG. 10(e) is a cross-sectional view cut along line R-R in FIG. 10(a).

As illustrated in FIG. 10(a), the mold 70 in this embodiment includes an internal cavity 71 for forming the balloon 13. The internal cavity 71 includes a cavity portion 71a, a cavity portion 71b, a cavity portion 71c, a cavity portion 71d, and a cavity portion 71e. The cavity portion 17a is for forming the base-end leg portion 13a of the balloon 13. The cavity portion 71b is for forming the base-end tapered portion 13b. The cavity portion 71c is for forming the straight tube portion 13c. The cavity portion 71d is for forming the tip-end tapered portion 13d. The cavity portion 71e is for forming the tip-end leg portion 13e. The cavity portion 71c for forming the straight tube portion 13c among the cavity portions 71a to 71e corresponds to "a cavity portion" recited in the claims.

As illustrated in FIGS. 10(c) and 10(d), the mold 70 includes a periphery wall 73 that defines the cavity portion 71c for the straight tube portion 13c. The periphery wall 73 includes protruding portions 75 that protrude inward on an inner peripheral side of the periphery wall 73. Each protruding portion 75 extends in a direction that is angled to the longitudinal direction of the internal cavity 71 (i.e., the axial direction of the internal cavity 71). Multiple (specifically three) protruding portions 75 are at equal intervals in the circumferential direction of the periphery wall 73. The protruding portions 75 of the periphery wall 73 extend for the entire length of the internal cavity 71 with respect to the longitudinal direction of the internal cavity 71. In FIG. 10(a), the mold 70 is illustrated in a simplified form without the protruding portions 75 for explanatory convenience (this also applied to protruding portions 79, 82 that will be described later).

The protruding portions 75 includes grooves 76. The grooves 76 extend along the protruding portions 75. Each groove 76 extends in a direction that is angled to the longitudinal direction of the internal cavity 71. Each groove 76 extends for an entire length of the internal cavity 71 that is defined by the periphery wall 73 with respect to the longitudinal direction.

As illustrated in FIG. 10(b), the mold 70 includes a periphery wall 78 that defines the cavity portion 71b for the base-end tapered portion 13b. The periphery wall 78 includes protruding portions 79 that protrude inward on an inner peripheral side of the periphery wall 78. The protruding portions 79 extend in the longitudinal direction of the internal cavity 71 and along a wall surface of the periphery wall 78. Multiple (specifically three) protruding portions 79 are at equal intervals in the circumferential direction of the periphery wall 78. Each protruding portion 79 extends for an entire length of the periphery wall 78 with respect to the longitudinal direction.

As illustrated in FIG. 10(e), the mold 70 includes a periphery wall 81 that defines the cavity portion 71d for the tip-end tapered portion 13d. The periphery wall 81 includes protruding portions 82 that protrude inward on an inner peripheral side of the periphery wall 81. The protruding portions 82 extend in the longitudinal direction of the internal cavity 71 and along the wall surface of the periphery wall 81. Multiple (specifically three) protruding portions 82 are at equal intervals in the circumferential direction of the periphery wall 81. Each protruding portion 82 extend for an entire length of the periphery wall 81 with respect to the longitudinal direction.

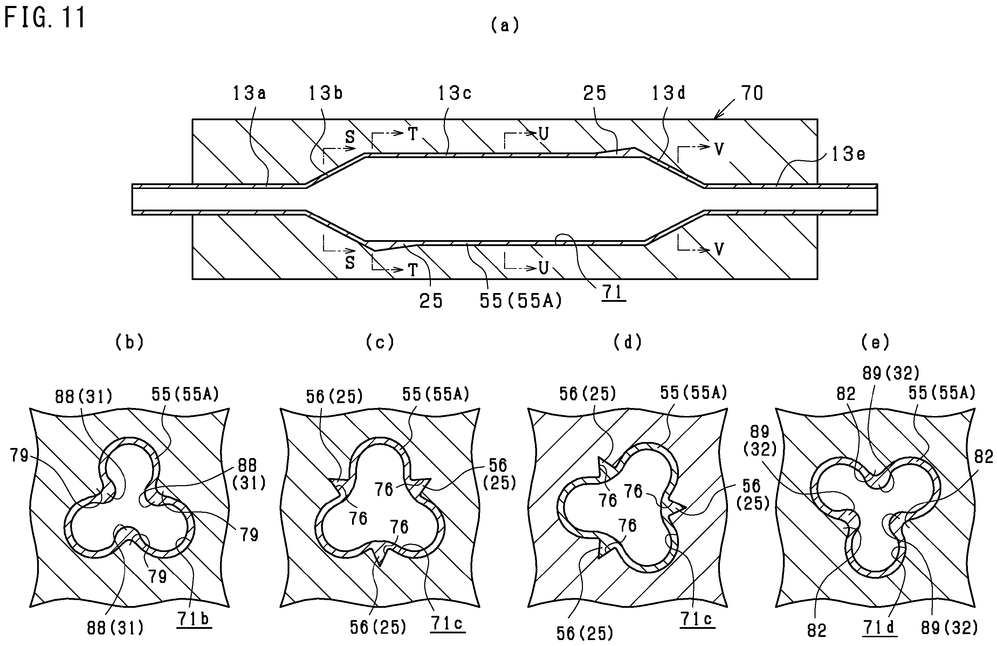

Next, a method for producing the balloon 13 using the mold 70 will be described with reference to FIGS. 11(a) to 11(e). FIG. 11(a) is a cross-sectional view illustrating an inflating step in this embodiment. FIG. 11(b) is a cross-sectional view cut along line S-S in FIG. 11(a). FIG. 11(c) is a cross-sectional view cut along line T-T in FIG. 11(a). FIG. 11(d) is a cross-sectional view cut along line U-U in FIG. 11(a). FIG. 11(e) is a cross-sectional view cut along line V-V in FIG. 11(a).

For producing the balloon 13, a preparing step, a placing step, a twisting step, a stretching step, an expanding step, and a cutting step are performed in sequence, similar to the first embodiment. In this embodiment, the external protrusions 25 and the internal protrusions 31, 32 can be easily formed in the expanding step because the mold 70 includes the protruding portions 75, 79, and 82. Such a point will be described below.

As illustrated in FIG. 11(a), in the expanding step, the expanded parison body 55A that includes the portions corresponding to the shapes of the balloon 13 is formed by thermally expanding the parison 55 that is twisted in the internal cavity 71 of the mold 70. As illustrated in FIGS. 11(c) and 11(d), in the expanding step, the external protrusions 25 are formed by inserting the protrusions 56 of the parison 55 into the grooves 76 of the mold 70. Because the grooves 76 are in the protruding portions 75 of the mold 70, the protrusions 56 are properly inserted in the grooves 76 during the expansion of the parison 55. This properly reduces displacement of the protrusions 56 from the grooves 76 during the expansion of the parison 55. Therefore, the external protrusions 25 are properly formed.

As illustrated in FIG. 11(b), in the expanding step, the protrusions 56 of the parison 55 are pressed against the protruding portions 79 of the mold 70, respectively, and smashed by expanding the parison 55 to cause portions 88 of the parison 55 along the protrusions 56 to protrude inward on the inner peripheral side to form the internal protrusions 31. According to this step, the portions 88 of the parison 55 along the protrusions 56 easily protrude inward on the inner peripheral side. Therefore, the internal protrusions 31 are easily formed.

As illustrated in FIG. 11(e), in the expanding step, the protrusions 56 of the parison 55 are pressed against the protruding portions 82 of the mold 70, respectively, and smashed by expanding the parison 55 to cause portions 89 of the parison 55 along the protrusions 56 to protrude inward on the inner peripheral side to form the internal protrusions 32. According to this step, the portions 89 of the parison 55 along the protrusions 56 easily protrude inward on the inner peripheral side. Therefore, the internal protrusions 32 are easily formed.

### [Other Embodiments]

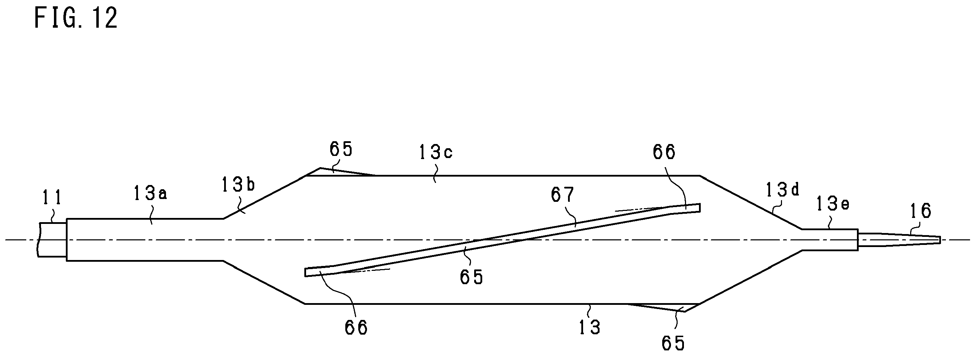

The present disclosure is not limited to the above embodiment and may be implemented as the following, for example.
(1) In the above embodiment, the angle of each external protrusion 25 relative to the axial direction of the straight tube portion 13c is in the range from 1 to 10 degrees. The angle of the external protrusion 25 can be greater than 10 degrees. If the angle is greater than 10 degree, each external protrusion 25 is more likely to be longer and the straight tube portion 13c is more likely to be thicker, which results in a reduction in insertability of the balloon 13. In view of this point, the angle of the external protrusion 25 is defined preferably in the range from 1 to 10 degrees.
(2) In the above embodiment, the angle of the each external protrusion 25 may be greater in each first portion 26 than in the second portion 27. Alternatively, as illustrated in FIG. 12, an angle of an external protrusion 65 may be less in each of two first portions 66 than in a second portion 67. According to the configuration, a pushing force for pushing the balloon 13 into a human body is more easily transmitted to the tip-end portion. The first portions 66 correspond to "first portions" recited in claim 5 and the second portion 67 corresponds to "a second portion" recited in claim 5.
(3) In the above embodiment, each external protrusion 25 includes two first portions 26 and the second portion 27, the angles of which are different from each other. Alternatively, the angle of the external protrusion 25 may be constant for an entire length of the external protrusion 25.
(4) In the above embodiment, the base-end tapered portion 13b and the tip-end tapered portion 13d include the internal protrusions 31, 32, respectively. Alternatively, only either one of the tapered portions 13b, 13d may include the internal protrusion or the tapered portions 13b, 13d may not include the internal protrusion.
(5) In the above embodiment, at least one of the tapered portions 13b, 13d may include a second external protrusion that protrudes from an outer periphery of the tapered portion. That is, the tapered portion may include the second external protrusion in addition to the internal protrusion. The second external protrusion may be formed to extend in the axial direction of the balloon 13 and along the outer periphery of the tapered portion. According to the configuration, cracks may be created in the lesion with the second external protrusion during the inflation of the balloon 13. Alternatively, the tapered portion may include the second external protrusion but not the internal protrusion.
(6) In the above embodiment, the stretching step is performed after the twisting step. Alternatively, the expanding step may be performed after the twisting step without the stretching step.
(7) In the above embodiment, the external protrusions 25 that are adjacent to each other in the circumferential direction of the balloon 13 are away from each other in the circumferential direction when viewed in the axial direction of the balloon 13. Alternatively, the external protrusions 25 that are adjacent to each other in the circumferential direction of the balloon 13 may be continuous in the circumferential direction when viewed in the axial direction of the balloon 13. Every two of the external protrusions 25 that are adjacent to each other may be continuous in the circumferential direction of the balloon 13 when viewed in the axial direction of the balloon 13 such that the external protrusions 25 may be present for an entire circumference of the balloon 13.
(8) Each internal protrusion 31 of the base-end tapered portion 13b may be formed such that the internal protrusion 31 shifts from the first side X to the second side Y in the circumferential direction of the balloon 13 from the base-end side to the tip-end side in the axial direction.
(9) In the configuration recited in (8), the internal protrusions 31 that are adjacent to each other in the circumferential direction of the balloon 13 may be continuous in the circumferential direction when viewed in the axial direction of the balloon 13. Every two of the internal protrusions 31 that are adjacent to each other may be continuous in the circumferential direction of the balloon 13 when viewed in the axial direction of the balloon 13 such that the internal protrusions 31 may be present for an entire circumference of the balloon 13.
(10) Each internal protrusion 32 of the tip-end tapered portion 13d may be formed such that the internal protrusion 32 shifts from the first side X to the second side Y in the circumferential direction of the balloon 13 from the base-end side to the tip-end side in the axial direction.
(11) In the configuration recited in (10), the internal protrusions 32 that are adjacent to each other in the circumferential direction of the balloon 13 may be continuous in the circumferential direction when viewed in the axial direction of the balloon 13. Every two of the internal protrusions 32 that are adjacent to each other may be continuous in the circumferential direction of the balloon 13 when viewed in the axial direction of the balloon 13 such that the internal protrusions 32 may be present for an entire circumference of the balloon 13.
(12) Any two of the configurations recited in (7), (9) and (11) may be employed or all three configurations may be employed. If the configurations recited in (7) and (11) are employed, the stretch of the straight tube portion 13c and the tip-end tapered portion 13d in the axial direction during the inflation of the balloon 13 is further suppressed.
(13) If the configuration recited in (10) is employed, the external protrusions 25 and the internal protrusions 32 may be disposed such that at least either of the external protrusions 25 and the internal protrusions 32 are present for the entire circumference of the balloon 13. According to the configuration, the stretch of the straight tube portion 13c and the tip-end tapered portion 13d in the axial direction during the inflation of the balloon 13 is further suppressed.
(14) If the configuration recited in (8) is employed, the external protrusions 25 and the internal protrusions 31 may be disposed such that at least either of the external protrusions 25 and the internal protrusions 31 are present for the entire circumference of the balloon 13.
(15) In the above embodiment, the balloon 13 that includes the external protrusions 25 is formed from the parison 55 that is formed by extrusion and has the same cross section. The parison 55 is not the only option for preparing the balloon 13. Another example of the parison may include protrusions that protrude from the outer periphery of the parison and extend in a direction that is angled to the longitudinal direction of the parison. If such a parison is used for preparing the balloon 13, the twisting step is not required. Therefore, the producing process of the balloon 13 can be shortened.
(16) During the expanding step, an extending step may be performed. In the extending step, the parison 55 that has been twisted in the twisting step may be extended in a direction in which the protrusions 56 of the parison 55 extend to align the molecular orientation of the expanded parison body 55A that has been formed in the expanding step or the balloon 13 in the longitudinal direction of the external protrusions 25. According to the step, stretch of the balloon 13 in the longitudinal direction of the external protrusions 25 is suppressed. Therefore, the balloon 13 is less likely to have a problem such as damages due to reaction from the lesion during the inflation of the balloon 13 to create cracks in the lesion with the external protrusions 25.
(17) The angle of the external protrusions 25 relative to the axial direction of the straight tube portion 13c when the balloon 13 is deflated may be less than the angle when the balloon 13 is inflated. According to the configuration, the wings 21 that cover the external protrusions 25 are more easily formed during the deflation of the balloon 13.

The present disclosure has been described with reference to the embodiments. However, the present disclosure is not limited to the embodiments or the structures. Various modifications and modifications within a scope of equivalents may be within in the technical scope of the present disclosure. Further, various combinations, configurations, and other combinations and configurations including single elements, more or less, may be within the technical scope of the present disclosure.

### Reference Sings List

10: Balloon catheter, 13: Balloon, 13b: Base tapered portion, 13c: Straight tube portion,13d: Tip tapered portion, 21: Wing, 25: External protrusion, 26: First portion, 27: Second portion, 31: Internal protrusion, 32: Internal protrusion, 40: Mold, 41: Internal cavity, 41c: Cavity portion, 43: Peripheral wall, 46: Groove, 55:Parison, 56: Protrusion, 70: Mold, 71: Internal cavity, 71c: Cavity portion, 75: Protruding portion, 76: Groove.

## Claims

1. A balloon catheter comprising a balloon that is inflatable and deflatable, wherein
the balloon includes a straight tube portion that is in a cylindrical shape with a diameter that is at a maximum when the balloon is inflated,
the straight tube portion includes an external protrusion that protrudes from an outer periphery of the straight tube portion, and
the external protrusion extends in a direction that is angled to an axial direction of the straight tube portion and along the outer periphery.

2. The balloon catheter according to claim 1, wherein an angle of the external protrusion relative to the axial direction is in a range from 1 to 10 degrees.

3. The balloon catheter according to claim 2, wherein
the balloon includes a wing that is formed when the balloon is deflated, and
the wing extends in the axial direction and is disposed to cover the external protrusion from an outer side.

4. The balloon catheter according to any one of claims 1 to 3, wherein
the external protrusion includes two first portions at ends of the external protrusion with respect to a longitudinal direction of the external protrusion and a second portion between the first portions, and
an angle of the external protrusion relative to the axial direction is greater in the first portions than in the second portion.

5. The balloon catheter according to any one of claims 1 to 3, wherein
the external protrusion includes two first portions at ends of the external protrusion with respect to a longitudinal direction of the external protrusion and a second portion between the first portions, and
an angle of the external protrusion relative to the axial direction is less in the first portions than in the second portion.

6. The balloon catheter according to any one of claims 1 to 3, wherein
the balloon includes two tapered portions that are disposed on sides of the straight tube portion with respect to the axial direction to sandwich the straight tube portion and have a diameter that decreases as a distance from the straight tube portion increases, and
a predefined one of the tapered portions includes an internal protrusion that protrudes from an inner periphery of the predefined one of the tapered portions and extends in the axial direction and along the inner periphery.

7. The balloon catheter according to claim 6, wherein
the external protrusion extends for an entire length of the straight tube portion with respect to the axial direction, and
the internal protrusion extends for an entire length of the predefined one of the tapered portions with respect to the axial direction and a position of the internal protrusion with respect to a circumferential direction of the balloon is equal to a positon of an end of the external protrusion on a side on which the predefined one of the tapered portions is located.

8. The balloon catheter according to any one of claims 1 to 3, wherein
the external protrusion has a cross section in an inverted V-shape and includes two side faces that are adjacent to each other across a vertex of the inverted V-shape,
a first side face of the side faces has a surface roughness greater than a surface roughness of a second side face of the side faces in a tip-end portion of the external protrusion on a tip-end side with respect to the axial direction, and
the second side face has a surface roughness greater than a surface roughness of the first side face in a base-end portion of the eternal protrusion on a base-end side with respect to the axial direction.

9. A method for producing the balloon catheter according to any one of claims 1 to 3, wherein
a parison that is in a tubular shape and a base material of the balloon includes a protrusion on an outer periphery of the parison, wherein the protrusion that is for forming the external protrusion extends in a longitudinal direction of the parison,
a mold for producing the balloon includes an internal cavity for forming the balloon, wherein
the internal cavity is in an elongated shape corresponding to a shape of the balloon and includes a cavity portion for forming the straight tube portion, and
the mold includes a periphery wall that defines the cavity portion, wherein the periphery wall includes a groove that extends in a direction that is angled to a longitudinal direction of the internal cavity, the method comprises:
a twisting step of twisting the parison in a circumferential direction of the parison that is placed in the internal cavity to cause the protrusion to extend in the direction that is angled to the longitudinal direction; and
an expanding step of expanding the parison that is twisted in the twisting step in the internal cavity to form an expanded parison body that includes a portion in a balloon shape, wherein
in the expanding step, the protrusion is inserted in the groove by expanding the parison to form the external protrusion.

10. The method according to claim 9 further comprising a stretching step of stretching the parison that is twisted in the twisting step in the longitudinal direction and holding the parison stretched, wherein
the expanding step is performed after the stretching step.

11. The method according to claim 9, wherein
the periphery wall includes a protruding portion that protrudes inward on an inner peripheral side of the periphery wall and extends in the direction that is angled, and
the groove is formed in the protruding portion and extends along the protruding portion.
